# FASCICULE DE BREVET EUROPEEN

(11) **EP 3 642 350 B1**
(45) Date de publication et mention de la délivrance du brevet: **08.09.2021**
(21) Numéro de dépôt: 18731149.3
(22) Date de dépôt: 21.06.2018
(51) Int. Cl.: C12Q 1/6886

(54) **MÉTHODES DE PRÉDICTION DU RISQUE DE DÉVELOPPER UN SARCOME RADIO-INDUIT**
VERFAHREN ZUR VORHERSAGE DES RISIKOS ZUR ENTWICKLUNG VON STRAHLENINDUZIERTEM SARKOME
METHODS FOR PREDICTING THE RISK OF DEVELOPING RADIO-INDUCED SARCOMA

(30) Priorité: 22.06.2017 FR 1755686
(43) Date de publication de la demande: 29.04.2020
(73) Titulaire: Université de Bourgogne, 21000 Dijon (FR); Centre Georges François Leclerc, 21000 Dijon (FR)
(72) Inventeur: MAINGON, Philippe, 21000 Dijon (FR); MIRJOLET, Céline, 21110 Magny Sur Tille (FR); BOIDOT, Romain, 21310 Arceau (FR); TRUNTZER, Caroline, 21000 Dijon (FR)
(74) Mandataire: Plasseraud IP
(86) Numéro de dépôt international: PCT/EP2018/066679
(87) Numéro de publication internationale: WO 2018/234512

(56) Documents cités:
- H NAKANISHI ET AL: "Mutation of the p53 gene in postradiation sarcoma.", LAB INVEST, vol. 78, no. 6, 1 juin 1998 (1998-06-01), pages 727-733, XP055418854,
- AMY BERRINGTON DE GONZALEZ ET AL: "Sarcoma risk after radiation exposure", CLINICAL SARCOMA RESEARCH, BIOMED CENTRAL LTD, LONDON, UK, vol. 2, no. 1, 4 octobre 2012 (2012-10-04) , page 18, XP021123054, ISSN: 2045-3329, DOI: 10.1186/2045-3329-2-18
- NATHALIE GONIN-LAURENT ET AL: "Specific TP53 mutation pattern in radiation-induced sarcomas", CARCINOGENESIS., vol. 27, no. 6, 20 février 2006 (2006-02-20), pages 1266-1272, XP055418877, GB ISSN: 0143-3334, DOI: 10.1093/carcin/bgi356
- NABILA-SANDRA HADJ-HAMOU ET AL: "A transcriptome signature distinguished sporadic from postradiotherapy radiation-induced sarcomas", CARCINOGENESIS., vol. 32, no. 6, 5 avril 2011 (2011-04-05), pages 929-934, XP055419039, GB ISSN: 0143-3334, DOI: 10.1093/carcin/bgr064 & Nabila-Sandra Hadj-Hamou ET AL: "A transcriptome signature distinguished sporadic from postradiotherapy radiation-induced sarcomas: Supplementary Tab. 4", , 24 janvier 2011 (2011-01-24), XP055419060, Extrait de l'Internet: URL:https://www.ncbi.nlm.nih.gov/pmc/artic les/PMC3314285/bin/supp_bgr064_Supp_Table_ 4.pdf [extrait le 2017-10-25]
- SAM BEHJATI ET AL: "Mutational signatures of ionizing radiation in second malignancies", NATURE COMMUNICATIONS, vol. 7, 12 septembre 2016 (2016-09-12), page 12605, XP055489965, DOI: 10.1038/ncomms12605

## Description

### Domaine technique de l'invention

La présente invention concerne des méthodes de prédiction du risque de développer un sarcome après un traitement par radiothérapie.

### Arrière-plan technologique

La radiothérapie constitue avec la chirurgie les deux piliers majeurs du traitement locorégional du cancer. Plus de 50% des patients atteints d'un cancer vont être pris en charge par radiothérapie. En 2015 ce nombre a représenté 204 471 patients (données INCa 2017) dont environ un quart était traité pour un cancer du sein. En France, 900 enfants sont pris en charge en radiothérapie chaque année.

Le risque d'apparition d'un second cancer suite à un traitement par radiothérapie devient mieux évalué et représente un souci majeur en termes de radioprotection. Parmi les lésions malignes découvertes chez des patients traités, l'apparition d'un sarcome est un évènement rare mais de pronostic sombre puisque le taux de survie des patients est estimé entre 10 à 35 % à 5ans. Les facteurs de risque de ce type d'apparition sont inconnus et les estimations de l'incidence de ce développement varient de 1% à 1,75% chez les patients irradiés survivants. En moyenne le développement de ces sarcomes est observé entre 5 et 10 ans après radiothérapie.

Nakanishi, et al. (LAB INVEST. 78.6 (1998): 727-733) divulguent que des mutations et des altérations dans le gène p53 sont très fréquemment observées après radiothérapie, et que ces modifications pourraient être liées à la carcinogénèse des sarcomes radio-induits.

Gonin-Laurent et al. (Carcinogenesis 27.6 (2006): 1266-1272) démontrent que les radiations induisent plusieurs altérations génétiques, notamment dans le gène TP53, et donc que les altérations dans le gène TP53 pourraient faire partir d'une signature génétique des sarcomes radio-induits.

Actuellement, les seules études conduites sur le risque de développer un cancer radio-induits étaient focalisées sur la dosimétrie avec la recherche des doses reçues sur les tissus où se développe le cancer (Berrington de Gonzalez et al. 2012, Clinical Sarcoma Research, 2: 18). Cependant, aucune étude n'a permis de découvrir d'éventuels facteurs génétiques impliqués dans le risque de développer un cancer induit par un traitement par radiothérapie.

Il existe donc un réel besoin d'identifier de nouveaux facteurs permettant d'évaluer avec exactitude les risques d'un patient de développer un cancer secondaire suite à un traitement par radiothérapie. L'évaluation de ce risque est essentielle pour assurer la meilleure prise en charge possible pour le patient, par exemple en lui évitant, si possible, le traitement par radiothérapie ou en lui assurant un suivi approfondi suite à son traitement lorsque le risque associé à l'apparition d'un second cancer est élevé.

### Description détaillée de l'invention

Les présents inventeurs sont parvenus à démontrer que la présence de variations nucléotidiques au sein de la séquence d'un groupe de gènes spécifique permettait de déterminer, avec une grande sélectivité et spécificité, le risque pour un patient de développer un risque de sarcome radio-induit. Ils ont particulièrement démontré que la détection d'une variation nucléotidique au sein de la séquence d'au moins 11 des gènes choisis dans le groupe constitué de KLRF1, C1orf234, PKD2L2, ZNF845, GRB2, TMEM117, KIRREL2, GCNT7, USP3, C1R, TOE1, QSER1, SCAMP1, SEPT7, SLC4A3, TBC1D10A, ZFP41, ZNF2, ICAM2, PRG4, MZT1, SYCE1, MAP1B, PRSS35, RPL11, JMJD4, SHISA4, FZD5, ISCU, LGI1, OSCAR, KDM4B, MIR3199-1, MIR3199-2, LIPI, ATP11C, ESF1, BCAS3, FAM63A, SNAP47, MUTYH, TTC28-AS1, RTFDC1 et SPRNP1, permettait de prédire le risque pour un patient de développer un cancer radio-induit.

Ainsi, selon un premier aspect, la présente description porte sur un procédé *in vitro* pour déterminer le risque de développer un sarcome radio-induit chez un patient traité par radiothérapie, ledit procédé comprenant une étape de détection, dans un échantillon biologique obtenu dudit patient, d'une variation nucléotidique dans la séquence d'au moins 11 gènes choisis parmi le groupe constitué de KLRF1, Clorf234, PKD2L2, ZNF845, GRB2, TMEM117, KIRREL2, GCNT7, USP3, C1R, TOE1, QSER1, SCAMP1, SEPT7, SLC4A3, TBC1D10A, ZFP41, ZNF2, ICAM2, PRG4, MZT1, SYCE1, MAP1B, PRSS35, RPL11, JMJD4, SHISA4, FZD5, ISCU, LGI1, OSCAR, KDM4B, MIR3199-1, MIR3199-2, LIPI, ATP11C, ESF1, BCAS3, FAM63A, SNAP47, MUTYH, TTC28-AS1, RTFDC1 et SPRNP1.

Les inventeurs ont particulièrement démontré que la présence d'une variation nucléotidique dans la séquence d'au moins 11 de ces gènes était témoin d'un risque accru de développer un cancer suite à un traitement par radiothérapie.

Ainsi, dans un autre aspect, l'invention porte sur un procédé *in vitro* pour déterminer le risque de développer un sarcome radio-induit chez un patient traité par radiothérapie, ledit procédé comprenant une étape de détection, dans un échantillon biologique obtenu dudit patient, d'une variation nucléotidique dans la séquence d'au moins 11 gènes choisis parmi le groupe constitué de KLRF1, Clorf234, PKD2L2, ZNF845, GRB2, TMEM117, KIRREL2, GCNT7, USP3, C1R, TOE1, QSER1, SCAMP1, SEPT7, SLC4A3, TBC1D10A, ZFP41, ZNF2, ICAM2, PRG4, MZT1, SYCE1, MAP1B, PRSS35, RPL11, JMJD4, SHISA4, FZD5, ISCU, LGI1, OSCAR, KDM4B, MIR3199-1, MIR3199-2, LIPI, ATP11C, ESF1, BCAS3, FAM63A, SNAP47, MUTYH, TTC28-AS1, RTFDC1 et SPRNP1, la présence d'une variation nucléotidique dans la séquence d'au moins 11 de ces gènes étant synonyme d'un risque accru pour le patient de développer un sarcome radio-induit.

Dans un autre mode de réalisation, pour réaliser le procédé selon la présente invention, une variation nucléotidique sera recherchée dans 12 des 44 gènes cités ci-dessus. Particulièrement, le procédé selon la présente invention peut comprendre la détection de variations nucléotidiques dans 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43ou 44 de ces gènes. Le procédé selon la présente invention peut également comprendre la détection d'une variation nucléotidique dans tous les 49 gènes selon la présente invention.

Dans le cadre de la présente invention, l'« échantillon biologique » peut être n'importe quel échantillon dans lequel de l'ADN humain peut être détecté, Typiquement, l'ADN humain pourra être détecté dans des lymphocytes provenant d'un prélèvement sanguin, d'un punch de peau, ou d'un prélèvement salivaire.

Tous les gènes selon l'invention sont connus en tant que tels et sont listés dans le tableau ci-dessous :

**Tableau A : Liste des gènes selon l'invention**

| Gene symbole | nom du Gene | Gene ID |
|---|---|---|
| ESF1 | ESF1, nucleolar pre-rRNA processing protein, homolog (S. cerevisiae) | ENSG00000089048 |
| PRSS35 | protease, serine, 35 | ENSG00000146250 |
| TMEM117 | transmembrane protein 117 | ENSG00000139173 |
| QSER1 | glutamine and serine rich 1 | ENSG00000060749 |
| SYCE1 | synaptonemal complex central element protein 1 | ENSG00000171772 |
| ZNF2 | zinc finger protein 2 | ENSG00000163067 |
| LIPI | lipase, member I | ENSG00000188992 |
| LGI1 | leucine-rich, glioma inactivated 1 | ENSG00000108231 |
| OSCAR | osteoclast associated, immunoglobulin-like receptor | ENSG00000170909 |
| MIR3199-1 | microRNA 3199-1 | ENSG00000264073 |
| SPRNP1 | shadow of prion protein homolog (zebrafish) pseudogene 1 | NA |
| SLC4A3 | soluté carrier family 4, anion exchanger, member 3 | ENSG00000114923 |
| JMJD4 | jumonji domain containing 4 | ENSG00000081692 |
| KDM4B | lysine (K)-specific demethylase 4B | ENSG00000127663 |
| TOE1 | target of EGR1, member 1 (nuclear) | ENSG00000132773 |
| ICAM2 | intercellular adhesion molecule 2 | ENSG00000108622 |
| SNAP47 | synaptosomal-associated protein, 47kDa | ENSG00000143740 |
| GCNT7 | glucosaminyl (N-acetyl) transferase family member 7 | ENSG00000124091 |
| MIR3199-2 | microRNA 3199-2 | ENSG00000283225 |
| ISCU | iron-sulfur cluster scaffold homolog (E. coli) | ENSG00000136003 |
| USP3 | ubiquitin specific peptidase 3 | ENSG00000140455 |
| MAP1B | microtubule-associated protein 1B | ENSG00000131711 |
| MUTYH | mutY homolog (E. coli) | ENSG00000132781 |
| SCAMP1 | secretory carrier membrane protein 1 | ENSG00000085365 |
| KIRREL2 | kin of IRRE like 2 (Drosophila) | ENSG00000126259 |
| MZT1 | mitotic spindle organizing protein 1 | ENSG00000204899 |
| RPL11 | ribosomal protein L11 | ENSG00000142676 |
| FZD5 | frizzled family receptor 5 | ENSG00000163251 |
| C1R | complément component 1, r subcomponent | ENSG00000159403 |
| PKD2L2 | polycystic kidney disease 2-like 2 | ENSG00000078795 |
| TTC28-AS1 | TTC28 antisense RNA 1 | ENSG00000235954 |
| ZNF845 | zinc finger protein 845 | ENSG00000213799 |
| TBC1D10A | TBC1 domain family, member 10A | ENSG00000099992 |
| SHISA4 | shisa homolog 4 (Xenopus laevis) | ENSG00000198892 |
| ATP11C | ATPase, class VI, type 11C | ENSG00000101974 |
| KLRF1 | killer cell lectin-like receptor subfamily F, member 1 | ENSG00000150045 |
| GRB2 | growth factor receptor-bound protein 2 | ENSG00000177885 |
| RTFDC1 | Replication Termination Factor 2 Domain Containing 1 | ENSG00000022277 |
| ZFP41 | Zinc Finger Protein | ENSG00000181638 |
| C1orf234 | Chromosome 1 Open Reading Frame 234 | ENSG00000227868 |
| PRG4 | Proteoglycan 4 | ENSG00000116690 |
| SEPT7 | Septin 7 | ENSG00000122545 |
| BCAS3 | breast carcinoma amplified sequence 3 | ENSG00000141376 |
| FAM63A | family with sequence similarity 63, member A | ENSG00000143409 |

Une « variation nucléotidique » correspond à une variation d'au moins un nucléotide dans la séquence d'un gène. Une variation d'au moins un nucléotide peut être :
- une délétion, c'est-à-dire la suppression d'au moins un nucléotide au sein de la séquence du gène
- une insertion, c'est-à-dire l'addition d'au moins un nucléotide dans la séquence du gène ;
- une substitution, c'est-à-dire le remplacement d'un nucléotide par une autre.

L'homme du métier connait un grand nombre de techniques lui permettant de détecter une variation de nucléotides au sein d'un gène.

Une variation nucléotidique est typiquement détectée chez un patient en comparant la séquence d'un acide nucléique exprimé par ledit patient avec la séquence de l'acide nucléique correspondant exprimé dans une population contrôle.

Selon la présente invention, la variation nucléotidique peut être détectée en analysant les molécules d'acides nucléiques des gènes selon l'invention. Ces molécules d'acides nucléiques sont de l'ADN génomique double brin.

Comme indiqué plus haut, la molécule d'acide nucléique est obtenue à partir de n'importe quel échantillon biologique contenant de l'ADN comme, par exemple, un échantillon de sang. L'ADN peut être extrait selon les méthodes décrites dans le manuel de Sambrook et al (1989). Un grand nombre de techniques peuvent être utilisées pour analyser un génotype (voir par exemple Antonarakis et al., 1989; Cooper et al., 1991; Grompe, 1993). Par exemple, on peut citer l'analyse du polymorphisme de longueur des fragments de restriction (RFLP) ; l'hybridation avec des oligonucléotides allèle-spécifiques qui sont des petites sondes synthétiques qui ne s'hybrident que lorsqu'il y a une parfaite identité de séquence dans des condition d'hybridation appropriées ; la PCR allèle-spécifique, la PCR avec des amorces mutagènes ; la discrimination allélique, l'analyse de fragments, la PCR HRM, la ligase-PCR ; l'électrophorèse sur gel en gradient dénaturant (DGGE); le polymorphisme de conformation des simples brins (SSCP) et la Chromatographie liquide à haute performance sur gel dénaturant (Kuklin et al., 1997).

Le séquençage direct peut être effectué par n'importe quelle méthode connue de l'Homme du métier comme par exemple le séquençage chimique selon la méthode de Maxam et de Gilbert ; le séquençage enzymatique selon la méthode de Sanger ; le séquençage par spectrométrie de masse ; et le séquençage de nouvelle génération, quelle que soit la technologie employée pour le séquençage (séquençage sur support solide, séquençage par nanopore, séquençage en pHmétrie...) ou pour la préparation de la librairie (capture ou amplicons).

Ainsi, des molécules d'acides nucléiques utiles à la présente invention sont par exemples des sondes ou des amorces. Ces sondes et amorces peuvent particulièrement s'hybrider aux régions où la mutation est localisée. Les sondes ou amorces nucléotidiques peuvent au moins contenir 10, 15, 20 ou 30 nucléotides. Leur longueur peut être inférieure à 400, 300, 200 ou 100 nucléotides.

Les variations de nucléotides selon la présente invention peuvent se situer dans les exons (c'est-à-dire dans les parties codantes du gène) mais également dans les introns (c'est-à-dire les régions non-codantes du gène). Ainsi, ces variations de nucléotides peuvent avoir un impact sur la protéine codée par le gène (mutations faux-sens, mutations non-sens), ou ne pas avoir de conséquence sur la séquence de la protéine (mutations silencieuses).

Dans un mode de réalisation particulier, la variation nucléotidique selon la présente invention se retrouve dans moins de 10% de la population générale, particulièrement moins de 5% de la population générale, et, de manière préférée, dans moins de 1% de la population générale. Typiquement le référentiel « population générale » utilise toutes les bases de données disponibles (exemple : 1000genomes, dbSNP, Ensembl, Exac...).

Dans le cadre de la présente description, le « cancer primaire », c'est-à-dire le cancer pour lequel le patient est traité par radiothérapie, est n'importe quel cancer qui peut être traité par ce genre de thérapie. Particulièrement, ce cancer primaire est choisi dans le groupe constitué du cancer du sein, d'un cancer des voies aérodigestives supérieures (cancer ORL), du cancer colorectal, du cancer du poumon, du cancer du col de l'utérus, du cancer de l'endomètre, du cancer des ovaires, du cancer de la prostate, du cancer des testicules, des tumeurs cérébrales ou du système nerveux central, du cancer de la thyroïde et du cancer ophtalmique. Dans un mode de réalisation particulier, ledit cancer primaire est un cancer du sein.

Le terme « cancer radio-induit » selon la présente description, signifie tout cancer induit suite à un traitement par radiothérapie. Dans un mode de réalisation particulier, le cancer est un sarcome, particulièrement un sarcome des tissus mous. La « radiothérapie » est un traitement locorégional du cancer consistant à utiliser des rayonnements pour détruire les cellules cancéreuses. En moyenne, les patients traités par radiothérapie reçoivent une dose de rayons de 50 à 80 Gray (Gy). Dans un mode de réalisation particulier, la méthode selon l'invention est réalisée avant le traitement du patient par radiothérapie. Selon ce mode de réalisation, il est donc possible de déterminer avant le traitement si le patient a de fortes chances de développer un cancer radio-induit suite à son traitement. Cela permet notamment, lorsque cela est possible, de changer de stratégie de traitement pour ce patient. Par exemple, on peut choisir de traiter ce patient par chirurgie, par chimiothérapie ou par immunothérapie plutôt que d'utiliser la radiothérapie.

Ainsi, dans un autre mode de réalisation, la présente description permet également d'élaborer une méthode de traitement d'un patient atteint d'un cancer, dans laquelle un patient ladite méthode comprenant les étapes suivantes :
a) détection, dans un échantillon biologique obtenu dudit patient, d'une variation nucléotidique dans la séquence d'au moins 11 gènes choisis parmi le groupe constitué de KLRF1, Clorf234, PKD2L2, ZNF845, GRB2, TMEM117, KIRREL2, GCNT7, USP3, C1R, TOE1, QSER1, SCAMP1, SEPT7, SLC4A3, TBC1D10A, ZFP41, ZNF2, ICAM2, PRG4, MZT1, SYCE1, MAP1B, PRSS35, RPL11, JMJD4, SHISA4, FZD5, ISCU, LGI1, OSCAR, KDM4B, MIR3199-1, MIR3199-2, LIPI, ATP11C, ESF1, BCAS3, FAM63A, SNAP47, MUTYH, TTC28-AS1, et SPRNP1.
b) détermination, au vu des résultats obtenus à l'étape a), du risque de développer un cancer radio-induit par ledit patient ;
c) traitement du patient par un autre traitement que la radiothérapie lorsque le risque déterminé à l'étape b) est accru.

Il est également possible que le patient ne puisse pas être traité par un autre traitement que la radiothérapie. Dans ce cadre, si la méthode selon la présente invention met en évidence un risque accru pour le patient de développer un cancer radio-induit, alors il pourra être décidé que le patient devra être suivi de manière plus poussée afin de pouvoir détecter au plus vite l'apparition d'un cancer radio-induit. Les patients développent en général un cancer radio-induit dans les 15 ans, voir les 10 ans et plus particulièrement dans les 5 à 10 ans qui suivent le traitement par radiothérapie. Ainsi, lorsque un patient qui a été traité ou qui va être traité par radiothérapie présente un risque de développer un cancer radio-induit selon la présente invention, alors ledit patient bénéficiera d'un suivi approfondi pendant les 5, 10 ou 15 années suivant son traitement. Ce suivi consistera par exemple à surveiller l'apparition d'un cancer, par exemple en mesurant régulièrement des biomarqueurs représentatifs d'un cancer donné. Par exemple, on pourra surveiller les biomarqueurs qui permettent de détecter et de suivre l'évolution des sarcomes. Ces marqueurs sont par exemple décrits dans Satelli et al (Cancer Res., 2014, 74(6), 1645-1650) ou dans Namlos et al (BMC Cancer, 2017, 17(1), 29). La méthode selon l'invention permet donc de détecter le plus tôt possible l'apparition d'un cancer radio-induit et donc une prise en charge précoce de ce dit cancer, ce qui augmente fortement les chances de survie du patient. Dans ce mode de réalisation, la méthode selon la présente invention peut-être réalisée avant ou après le traitement du patient par radiothérapie.

Selon la présente description, un « patient » est un humain. Dans un mode de réalisation particulier, le patient est un patient de moins de 18 ans. En effet, comme indiqué plus haut, environ 900 enfants sont traités par radiothérapie chaque année en France. Après le traitement de leur cancer primaire, ces enfants ont encore une longue durée de vie devant eux. L'impact du traitement sur la durée de vie de ces enfants à long terme est donc une question de haute importance. L'utilisation de la méthode selon l'invention s'avère donc particulièrement avantageuse chez ce type de patients.

La présente invention est présentée de manière plus détaillée dans les exemples ci-dessous. Ces exemples sont fournis uniquement à des fins illustratives et ne peuvent être interprétés comme limitant la portée de la présente invention. La présente invention est définie dans les revendications.

### EXEMPLES

### MATERIEL ET METHODES :

Nous démontrons ici que la détection d'une variation nucléotidique au sein d'au moins 11 des gènes présentés dans le Tableau A permet de déterminer avec précision le risque d'un patient de développer un cancer radio-induit suite à un traitement par radiothérapie. Les résultats démontrent que la détection de variations nucléotidiques au sein de ces gènes permet de prédire un cancer radio-inuit chez plus de 80% des patients.

### Patients et échantillons :

L'ensemble des résultats ayant permis de générer cette signature provient de l'étude SARI (étude des paramètres prédictifs de l'apparition de sarcomes développés en territoire irradié). L'objectif primaire de cette étude était de rechercher une signature génétique pouvant prédire une prédisposition d'un patient à développer un second cancer de type sarcome après avoir été pris en charge par radiothérapie pour un premier cancer. Cette étude cas/témoins a nécessité l'inclusion de 360 patients : 120 patients CAS ayant développé un sarcome radio-induit et 240 patients TEMOINS ayant reçu de la radiothérapie depuis plus de 5 ans et n'ayant pas développé de sarcome secondaire. Les CAS / TEMOINS étaient appariés sur plusieurs critères : l'âge du patient au moment de la radiothérapie initiale, le sexe et la localisation de la tumeur primaire.

Un prélèvement sanguin de 8 ml a été réalisé pour l'ensemble des patients en tubes EDTA. Les lymphocytes ont été récupérés par une technique de FICOL puis les ADN extraits.

Les exomes ont ensuite été préparés avec le kit SureSelect XT Human All Exon v5 (Agilent) et séquencés sur un séquenceur NextSeq500 (Illumina). Les data générés ont été prétraités par un processus Illumina/Annotations Variant Studio (profondeur de lecture>10, fréquence de l'allèle alternatif>30, Filtre PASS). Les variants ont ensuite été subdivisés en deux catégories : Variants synonymes et Variants introniques/codants.

L'analyse statistique a été réalisée en multivariée. Différentes méthodes de sélection de variables en grande dimension ont été utilisées (Sparse PLS, lasso, forêts aléatoires). L'ensemble des variables sélectionnées par ces différentes approches a été retenu et a permis de sélectionner 44 variables*. Une méthode de sélection de variables basée sur des forêts aléatoires en situation de grande dimension a ensuite été utilisée sur ce sous-ensemble de variables, dans l'objectif d'en conserver un nombre restreint. Cette méthode a permis de mettre en évidence les 44 gènes du Tableau A dont des modifications corrèlent avec une prédisposition des patients à développer un sarcome radio-induit.

## Revendications

1. Procédé *in vitro* pour déterminer le risque de développer un sarcome radio-induit chez un patient traité par radiothérapie, ledit procédé comprenant une étape de détection, dans un échantillon biologique obtenu dudit patient, d'une variation nucléotidique dans la séquence d'au moins 11 gènes choisis parmi le groupe constitué de KLRF1, Clorf234, PKD2L2, ZNF845, GRB2, TMEM117, KIRREL2, GCNT7, USP3, C1R, TOE1, QSER1, SCAMP1, SEPT7, SLC4A3, TBC1D10A, ZFP41, ZNF2, ICAM2, PRG4, MZT1, SYCE1, MAP1B, PRSS35, RPL11, JMJD4, SHISA4, FZD5, ISCU, LGI1, OSCAR, KDM4B, MIR3199-1, MIR3199-2, LIPI, ATP11C, ESF1, BCAS3, FAM63A, SNAP47, MUTYH, TTC28-AS1, et SPRNP1.

2. Procédé selon la revendication 1, dans laquelle la présence d'une variation nucléotidique dans la séquence d'au moins 11 gènes parmi KLRF1, Clorf234, PKD2L2, ZNF845, GRB2, TMEM117, KIRREL2, GCNT7, USP3, C1R, TOE1, QSER1, SCAMP1, SEPT7, SLC4A3, TBC1D10A, ZFP41, ZNF2, ICAM2, PRG4, MZT1, SYCE1, MAP1B, PRSS35, RPL11, JMJD4, SHISA4, FZD5, ISCU, LGI1, OSCAR, KDM4B, MIR3199-1, MIR3199-2, LIPI, ATP11C, ESF1, BCAS3, FAM63A, SNAP47, MUTYH, TTC28-AS1, et SPRNP1 est synonyme d'un risque accru pour le patient de développer un sarcome radio-induit.

3. Procédé selon la revendication 1 ou 2, dans lequel l'échantillon biologique est un échantillon de sang.

4. Procédé selon l'une quelconque des revendications 1 à 3, dans lequel ladite variation nucléotidique est présente dans moins de 1% de la population générale.

5. Procédé selon l'une quelconque des revendications 1 à 4, dans lequel l'échantillon est obtenu avant traitement dudit patient par radiothérapie.

6. Procédé selon l'une quelconque des revendications 1 à 4, dans lequel l'échantillon est obtenu après traitement dudit patient par radiothérapie.

7. Procédé selon l'une quelconque des revendications 1 à 6, dans lequel l'échantillon est obtenu à partir d'un patient âgé de moins de 18 ans.

## Patentansprüche

1. In-vitro-Verfahren zur Bestimmung des Risikos der Entwicklung eines strahleninduzierten Sarkoms bei einem mit Strahlentherapie behandelten Patienten, wobei das Verfahren einen Schritt des Nachweises einer Nukleotidvariation in der Sequenz von mindestens 11 Genen, gewählt aus der Gruppe, umfassend KLRF1, C1orf234, PKD2L2, ZNF845, GRB2, TMEM117, KIRREL2, GCNT7, USP3, C1R, TOE1, QSER1, SCAMP1, SEPT7, SLC4A3, TBC1D10A, ZFP41, ZNF2, ICAM2, PRG4, MZT1, SYCE1, MAP1B, PRSS35, RPL11, JMJD4, SHISA4, FZD5, ISCU, LGI1, OSCAR, KDM4B, MIR3199-1, MIR3199-2, LIPI, ATPI1C, ESF1, BCAS3, FAM63A, SNAP47, MUTYH, TTC28-AS1, RTFDC1 und SPRNP1 in einer von dem Patienten erhaltenen biologischen Probe umfasst.

2. Verfahren nach Anspruch 1, wobei das Vorliegen einer Nukleotidvariation in der Sequenz von mindestens 11 Genen aus KLRF1, Clorf234, PKD2L2, ZNF845, GRB2, TMEM117, KIRREL2, GCNT7, USP3, C1R, TOE1, QSER1, SCAMP1, SEPT7, SLC4A3, TBC1D10A, ZFP41, ZNF2, ICAM2, PRG4, MZT1, SYCE1, MAP1B, PRSS35, RPL11, JMJD4, SHISA4, FZD5, ISCU, LGI1, OSCAR, KDM4B, MIR3199-1, MIR3199-2, LIPI, ATP11C, ESF1, BCAS3, FAM63A, SNAP47, MUTYH, TTC28-AS1, RTFDC1 und SPRNP1 gleichbedeutend ist mit einem erhöhten Risiko für den Patienten, ein strahleninduziertes Sarkom zu entwickeln.

3. Verfahren nach Anspruch 1 oder 2, wobei die biologische Probe eine Blutprobe ist.

4. Verfahren nach einem der Ansprüche 1 bis 3, wobei die Nukleotidvariation in weniger als 1 % der allgemeinen Bevölkerung vorhanden ist.

5. Verfahren nach einem der Ansprüche 1 bis 4, wobei die Probe vor der Behandlung des Patienten mit Strahlentherapie erhalten wird.

6. Verfahren nach einem der Ansprüche 1 bis 4, wobei die Probe nach der Behandlung des Patienten mit Strahlentherapie erhalten wird.

7. Verfahren nach einem der Ansprüche 1 bis 6, wobei die Probe von einem Patienten im Alter unter 18 Jahren gewonnen wird.

## Claims

1. An *in vitro* method for determining the risk of developing a radio-induced sarcoma in a patient treated with radiotherapy, said method comprising a step of detecting, in a biological sample obtained from said patient, a nucleotide change in the sequence of at least 11 genes selected from the group consisting of KLRF1, Clorf234, PKD2L2, ZNF845, GRB2, TMEM117, KIRREL2, GCNT7, USP3, C1R, TOE1, QSER1, SCAMP1, SEPT7, SLC4A3, TBC1D10A, ZFP41, ZNF2, ICAM2, PRG4, MZT1, SYCE1, MAP1B, PRSS35, RPL11, JMJD4, SHISA4, FZD5, ISCU, LGI1, OSCAR, KDM4B, MIR3199-1, MIR3199-2, LIPI, ATP11C, ESF1, BCAS3, FAM63A, SNAP47, MUTYH, TTC28-AS1, and SPRNP1.

2. The method according to claim 1, wherein the presence of a nucleotide change in the sequence of at least 11 genes among KLRF1, C1orf234, PKD2L2, ZNF845, GRB2, TMEM117, KIRREL2, GCNT7, USP3, C1R, TOE1, QSER1, SCAMP1, SEPT7, SLC4A3, TBC1D10A, ZFP41, ZNF2, ICAM2, PRG4, MZT1, SYCE1, MAP1B, PRSS35, RPL11, JMJD4, SHISA4, FZD5, ISCU, LGI1, OSCAR, KDM4B, MIR3199-1, MIR3199-2, LIPI, ATP11C, ESF1, BCAS3, FAM63A, SNAP47, MUTYH, TTC28-AS1, and SPRNP1 is synonymous with an increased risk for the patient of developing a radio-induced sarcoma.

3. The method according to claim 1 or 2, wherein the biological sample is a blood sample.

4. The method according to any one of claims 1 to 3, wherein said nucleotide change is present in less than 1% of the general population.

5. The method according to any one of claims 1 to 4, wherein the sample is obtained before treatment of said patient by radiotherapy.

6. The method according to any one of claims 1 to 4, wherein the sample is obtained after treatment of said patient by radiotherapy.

7. The method according to any one of claims 1 to 6, wherein the sample is obtained from a patient under 18 years of age.
